Europäisches Patentamt

European Patent Office

Office européen des brevets

(11) Veröffentlichungsnummer: **0 315 020 B1**

# EUROPÄISCHE PATENTSCHRIFT

(45) Veröffentlichungstag der Patentschrift: **18.03.92**

(51) Int. Cl.⁵: **C07D 251/34**, C09D 175/16

(21) Anmeldenummer: **88117704.2**

(22) Anmeldetag: **25.10.88**

(54) **Verfahren zur Herstellung von Isocyanuratgruppen und olefinische Doppelbindungen aufweisenden Verbindungen und ihre Verwendung als Bindemittel.**

(30) Priorität: **03.11.87 DE 3737244**

(43) Veröffentlichungstag der Anmeldung:
**10.05.89 Patentblatt 89/19**

(45) Bekanntmachung des Hinweises auf die
Patenterteilung:
**18.03.92 Patentblatt 92/12**

(84) Benannte Vertragsstaaten:
**BE CH DE ES FR GB IT LI SE**

(56) Entgegenhaltungen:
**US-A- 4 128 537**
**US-A- 4 145 544**
**US-A- 4 159 376**
**US-A- 4 326 057**

(73) Patentinhaber: **BAYER AG**

**W-5090 Leverkusen 1 Bayerwerk(DE)**

(72) Erfinder: **Meixner, Jürgen, Dr.**
**Josef-Brocker-Dyk 56**
**W-4150 Krefeld(DE)**
Erfinder: **Bock, Manfred, Dr.**
**15143 Sewickly**
**311, Myrtle Lane(US)**
Erfinder: **Pedain, Josef, Dr.**
**Haferkamp 6**
**W-5000 Köln 80(DE)**
Erfinder: **Schönfelder, Manfred, Dr.**
**Höhenstrasse 126**
**W-5090 Leverkusen 3(DE)**

EP 0 315 020 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein neues Verfahren zur Herstellung von Isocyanuratgruppen und olefinische Doppelbindungen aufweisenden Verbindungen, die zur Herstellung von Beschichtungen mit verbesserten lacktechnischen Eigenschaften geeignet sind, und die Verwendung der neuen Verbindungen als Bindemittel.

Urethan(meth)acrylate, ihre Herstellung durch Umsetzung von organischen Polyisocyanaten mit Hydroxyalkyl(meth)acrylaten und ihre Verwendung als Bindemittel für UV-oder Elektronenstrahl-härtbare Beschichtungsmittel sind bekannt (vgl. z.B. DE-OS 1 644 779, DE-OS 2 115 373, DE-OS 2 734 237, DE-OS 3 118 147 oder GB-PS 1 491 695).

Die der Erfindung zugrundeliegende Aufgabe bestand darin, solche Urethan(meth)acrylate zur Verfügung zu stellen, die in Abwesenheit von Luftsauerstoff auch peroxidisch ausgehärtet werden können, und die gleichzeitig den Systemen des genannten Standes der Technik bezüglich der lacktechnischen Eigenschaften der resultierenden Beschichtungen mindestens ebenbürtig sind.

Diese Aufgabe konnte durch die Bereitstellung des nachstehend näher beschriebenen erfindungsgemäßen Verfahrens gelöst werden.

Gegenstand der Erfindung ist ein Verfahren zur Herstellung von Isocyanuratgruppen und olefinische Doppelbindungen aufweisenden Verbindungen durch Umsetzung von

a) einer, Isocyanuratgruppen aufweisende Polyisocyanate enthaltenden, Polyisocyanat-Komponente

mit

b) einer olefinisch ungesättigten Alkoholkomponente, bestehend aus mindestens einem Hydroxyalkylester der Acrylsäure oder der Methacrylsäure, dadurch gekennzeichnet, daß man

a) als Polyisocyanatkomponente (i) gegebenenfalls im Gemisch mit seinen höheren, mehr als einem Isocyanuratring aufweisenden Homologen vorliegendes $N,N',N''$-Tris-(isocyanatohexyl)isocyanurat oder (ii) Gemische der unter (i) genannten Polyisocyanate mit bis zu 40 NCO-Äquivalent-%, bezogen auf die gesamte Komponente a) an anderen Polyisocyanaten mit aliphatisch und/oder cycloaliphatisch gebundenen Isocyanatgruppen

verwendet und die Umsetzung unter Mitverwendung von

c) einer Polyolkomponente, bestehend im wesentlichen aus einem Polyesterpolyol der OH-Zahl 80 bis 350 auf Basis von (i) einer Säurekomponente, bestehend zu mindestens 80 Carboxyl-Äquivalent-% aus Adipinsäure und/oder Isophthalsäure und (ii) einer Polyolkomponente, bestehend zumindest zu 70 Hydroxyläquivalent-% aus 1,6-Hexandiol

durchführt, wobei die Menge der Komponente c) 20 bis 150 Gew.-%, bezogen auf das Gewicht der Komponente b) ausmacht und die Umsetzung unter Einhaltung eines NCO/OH-Äquivalentverhältnisse von 0,9:1 bis 1,1:1 durchführt, wobei die alkoholischen Komponenten b) und c) mit der Polyisocyanatkomponente in beliebiger Reihenfolge oder in Abmischung umgesetzt werden.

Gegenstand der Erfindung ist auch die Verwendung der nach diesem Verfahren erhaltenen Isocyanuratgruppen und olefinische Doppelbindungen aufweisenden Verbindungen als Bindemittel für unter dem Einfluß von Strahlen oder von Peroxiden in Gegenwart von Luftsauerstoff härtbaren Beschichtungsmitteln.

Die beim erfindungsgemäßen Verfahren einzusetzende Polyisocyanatkomponente a) besteht zu mindestens 60 NCO-Äquivalent-% aus $N,N',N''$-Tris-(6-isocyanatohexyl)-isocyanurat bzw. aus dessen Gemischen mit seinen höheren, mehr als einen Isocyanuratring aufweisenden Homologen. Die Herstellung derartiger Trimerisate des 1,6-Diisocyanatohexans wird beispielsweise in EP-A-10 589 oder US-PS 4 324 879 beschrieben. In den genannten Gemischen liegen im allgemeinen bis zu 50 Gew.-%, bezogen auf Gemisch, an höheren Homologen der genannten Art vor. Die Polyisocyanatkomponente a) kann bis zu 40 NCO-Äquivalent-% an anderen Polyisocyanaten mit aliphatisch und/oder cycloaliphatisch gebundenen Isocyanatgruppen enthalten. Bei diesen anderen Polyisocyanaten handelt es sich beispielsweise um 1,6-Diisocyanatohexan, 1-Isocyanato-3,3,5-trimethyl-5-isocyanatomethyl-cyclohexan (IPDI), Isocyanuratgruppen aufweisende Polyisocyanate auf Basis von IPDI oder Urethangruppen aufweisende Polyisocyanate auf Basis von IPDI und mehrwertigen Alkoholen wie beispielsweise Trimethylolpropan. Vorzugsweise besteht die Polyisocyanatkomponente a) zu mindestens 95 NCO-Äquivalent-% aus $N,N',N''$-Tris-(6-isocyanatohexyl)-isocyanurat oder dessen, obengenannten, Gemischen mit seinen höheren Homologen.

Bei der Alkoholkomponente b) handelt es sich um mindestens einen Hydroxyalkylester der Acrylsäure oder der Methacrylsäure, d.h. um einfache Ester dieser Säuren mit zweiwertigen aliphatischen Alkoholen des Molekulargewichtsbereichs 62 bis 300. Geeignete derartige Alkohole sind Ethylenglykol, 1,2-Propandiol, 1,3-Propandiol, die isomeren Butandiole, Pentandiole oder Hexandiole, oder beispielsweise Estergruppen aufweisende Diole auf Basis dieser einfachen Alkandiole und zweibasischen Säuren wie beispielsweise Adipinsäure, sofern die Ester innerhalb des genannten Molekulargewichtsbereichs liegen. Vorzugsweise

2

handelt es sich bei der Komponente b) um 2-Hydroxyethylacrylat, 2-Hydroxyethylmethacrylat, 2-Hydroxypropylacrylat, 2-Hydroxypropylmethacrylat oder um beliebige Gemische dieser Verbindungen.

Bei der Komponente c) handelt es sich um Polyesterpolyole des OH-Zahlbereichs 80 bis 350, die durch Umsetzung von (i) 33 bis 46 Mol-% einer Dicarbonsäurekomponente mit (ii) 54 bis 67 Mol-% einer Polyolkomponente in an sich bekannter Weise hergestellt worden sind.

Die Dicarbonsäurekomponente besteht zu mindestens 80 Carboxyl-Äquivalent-% aus Adipinsäure und/oder Isophthalsäure, vorzugsweise zu 0 bis 80 Mol-% aus Isophthalsäure und zu 20 bis 100 Mol-% aus Adipinsäure, wobei neben diesen beiden Säuren auch noch untergeordnete Mengen an anderen Dicarbonsäuren bzw. Dicarbonsäurederivaten mitverwendet werden können, wie beispielsweise Terephthalsäure, Phthalsäure, Phthalsäureanhydrid, Tetrahydrophthalsäure, Tetrahydrophthalsäureanhydrid, Hexahydrophthalsäure und/oder Hexahydrophthalsäureanhydrid. Die oben gemachten Angaben bezüglich der Carboxyläquivalentprozente beziehen sich auf die Gesamtmenge der zur Herstellung der Komponente c) eingesetzten Dicarbonsäuren bzw. Dicarbonsäurederivate, wobei die beispielhaft genannten Anhydride als "Dicarbonsäure" in die Berechnung eingehen.

Die Polyolkomponente (ii) besteht aus den üblichen, mehrwertigen Alkoholen, beispielsweise jenen, die oben bereits beispielhaft im Zusammenhang mit der Komponente b) genannt sind, wobei in der Komponente (ii) auch höherfunktionelle Alkohole wie Glycerin oder Trimethylolpropan vorliegen können, mit der Maßgabe, daß die Komponente (ii) zur Herstellung der Komponente c) zumindest zu 70 Hydroxyläquivalent-% aus 1,6-Hexandiol besteht.

Besonders bevorzugt wird als Komponente c) ein Polyesterdiol aus 1,6-Hexandiol und Adipinsäure verwendet.

Die Säurezahlen der Komponente c) liegen im allgemeinen im Bereich von 1,0 bis 10, daß aus der Stöchiometrie der eingesetzten Ausgangsmaterialien berechenbare Molekulargewicht bei 250 bis 3000.

Die Komponente c) wird im allgemeinen in einer Menge von 20 bis 150, vorzugsweise 20 bis 100 Gew.-%, bezogen auf das Gewicht der Komponente b) verwendet.

Zur Durchführung des erfindungsgemäßen Verfahrens werden die Komponenten b) und c) in beliebiger Reihenfolge oder in Form eines vorab hergestellten Gemischs aus den beiden Einzelkomponenten mit der Polyisocyanatkomponente a) zur Umsetzung gebracht, wobei das NCO/OH-Äquivalentverhältnis bei 0,9:1 bis 1,1:1, vorzugsweise bei ca. 1:1 liegt. Die Umsetzung erfolgt im allgemeinen innerhalb des Temperaturbereichs von 40 bis 100, vorzugsweise 50 bis 80°C, wobei jedoch darauf zu achten ist, daß unerwünschte thermisch induzierte Polymerisationsreaktionen unterbleiben. Es ist daher oft zweckmäßig, bei relativ niedrigen Temperaturen innerhalb der genannten Temperaturbereiche und unter Verwendung von bekannten, die Isocyanat-Additionsreaktion beschleunigenden Katalysatoren, zu arbeiten. Geeignete Katalysatoren sind beispielsweise Alkalimetallalkoholate wie Natrium-ethylat, tertiäre Amine wie Triethylamin, Diethylentriamin oder Dimethylbenzylamin oder beispielsweise bekannte Zinn-Katalysatoren wie Zinndioctoat oder Dibutylzinn-dilaurat.

Die Umsetzung kann in Substanz oder in Gegenwart von inerten Lösungsmitteln wie z.B. Ethylacetat, Butylacetat, Ethylglykolacetat und/oder Methylisobutylketon erfolgen.

Um die erfindungsgemäßen Urethanacrylate vor unerwünschter vorzeitiger Polymerisation zu bewahren, ist es oft empfehlenswert, bereits bei der Herstellung 0,001 bis 0,3 Gew.-%, bezogen auf das Gewicht der Ausgangsmaterialien, an bekannten Polymerisationsinhibitoren oder Antioxidantien wie z.B. die für diesen Zweck üblicherweise eingesetzten Chinone, Hydrochinone, Kupferverbindungen, Phosphite, Amine oder Phenole mitzuverwenden.

Die erfindungsgemäßen Produkte stellen im allgemeinen klare, mittel- bis hochviskose und farblose Flüssigkeiten dar.

Ist ihre Viskosität für die gewünschte Anwendung zu hoch, so können sie mit Lösungsmitteln verdünnt werden. Hierzu können aromatische Kohlenwasserstoffe, wie z.B. Toluol, Xylol und höher substituierte Benzole, Ester, wie etwa Ethylacetat, Butylacetat, Methoxy- oder Ethoxyethylacetat, Butoxyethylacetat, und Ketone wie Aceton, Methylethylketon, Methylisobutylketon oder Cyclohexan, aber auch Alkohole, wie Methanol, Ethanol, propanol, i-Propanol, Butanol, i-Butanol usw. herangezogen werden.

Im weiteren Sinne als Lösungsmittel können auch ethylenisch ungesättigte niedermolekulare Verbindungen angesehen werden, wie etwa Ester der Acryl- oder Methacrylsäure, aromatische Vinylverbindungen oder Vinylalkylether.

Diese ethylenisch ungesättigten "Lösungsmittel" stellen in Wirklichkeit keine echten Lösungsmittel dar sondern sind vielmehr als "reaktive Verdünner" anzusehen, da sie bei der Vernetzung der erfindungsgemäßen Produkte mit diesen unter Copolymerisation abreagieren.

Die erfindungsgemäßen Produkte stellen wertvolle Bindemittel für Überzüge dar. Sie können als solche oder in Kombination mit den aus der Lacktechnologie bekannten Hilfs- und Zusatzmitteln, wie z.B.

Füllstoffen, Pigmenten, Lösungsmitteln, Verlaufshilfsmitteln u.dgl. zur Herstellung von Beschichtungen auf beliebigen Substraten verwendet werden. Geeignete Substrate sind Papier, Kartonage, Leder, Holz, Kunststoffe, Vlies, Textilien, keramische Materialien, mineralische Materialien, Glas, Metall, Kunstleder, fotografische Materialien wie z.B. mit fotografischer Schicht versehenes Papier.

Der Auftrag dieser Beschichtungsmittel kann auf bekannte Weise durch Spritzen, Rakeln, Walzen, Streichen, Tauchen oder Gießen erfolgen. Nach Verdunsten von gegebenenfalls mitverwendeten inerten Lösungsmitteln kann die Vernetzung der Überzüge entweder mittels energiereicher Strahlung, wie UV-Licht, Elektronen oder Gammastrahlen oder durch Härtung mit Metallsalzen von Sikkativsäuren und (Hydro)-Peroxiden bei Temperaturen zwischen Raumtemperatur und 150°C erfolgen.

Im Falle der Vernetzung mittels UV-Bestrahlung ist es notwendig, der Überzugsmasse Fotoinitiatoren hinzuzufügen.

Als Fotoinitiatoren sind die üblicherweise eingesetzten Verbindungen geeignet, wie sie z.B. in der Monographie von J. Korsar, "Light-Sensitive-Systems", J. Wiley & Sons, New York - London - Sydney 1965 beschrieben sind.

Weiterhin gut geeignet sind Benzoinether wie Benzoinisopropylether, Benzilketale, wie z.B. Benzildimethylketal und Hydroxyalkylphenone, wie z.B. 2-Hydroxy-2-methyl-1-phenyl-propan-1-on.

Die erwähnten Fotoinitiatoren, die je nach Verwendungszweck der erfindungsgemäßen Massen in Mengen zwischen 0,1 bis 5 Gew.-%, bezogen auf polymerisierbare Komponenten, eingesetzt werden, können als einzelne Substanz oder, wegen häufiger vorteilhafter synergistischer Effekte, auch in Kombination miteinander verwendet werden.

Die bei der Vernetzung mittels Peroxiden der erfindungsgemäßen Urethanacrylate eingesetzten Metallsalze von Sikkativsäuren sind z.B. Kobalt-, Blei- und Mangansalze von Säuren wie Leinölfettsäuren, Tallölfettsäure, Sojaölfettsäuren, von Harzsäuren wie Abietinsäure und Naphthensäure oder von Essigsäure und Isooctansäure. Sie werden in Form von organischen Lösungen in solchen Mengen eingesetzt, daß der Metallgehalt, bezogen auf Urethanacrylat, 0,005 bis 1 Gew.-% entspricht.

Als (Hydro)-Peroxide seien beispielshaft genannt: Di-tert.-butylperoxid, Benzoylperoxid, Cyclohexanonperoxid, Methylethylketonperoxid, Acetylacetonperoxid, Dinonylperoxid, Bis-(4-tert.-butylcyclohexyl)-peroxydicarbonat, tert.-Butylhydroperoxid, Cumolhydroperoxid, 2,5-Di-methylhexan-2,5-hydroperoxid und Diisopropyl-benzol-monohydroperoxid. Vorzugsweise werden diese (Hydro)-Peroxide in Mengen von 1-10 Gew.-%, bezogen auf Urethanacrylat, eingesetzt.

In den nachfolgenden Beispielen beziehen sich alle Angaben in % auf Gewichtsprozente.

Beispiele

In den nachfolgenden Beispielen werden folgende Ausgangsmaterialien eingesetzt:

Polyisocyanat I

(Trimerisiertes 1,6-Diisocyanatohexan (HDI))

1344 g HDI werden bei 23°C mit 1 ml 2-Dimethylaminomethyl-nonylphenol versetzt. Nach 5-minütigem Verrühren tropft man ebenfalls bei 23°C innerhalb von 15 Minuten 40 ml einer 2 %igen Lösung von 2-Hydroxyethyl-trimethylammoniumhydroxid in Dimethylformamid/Methanol (8:1) ein.

Innerhalb dieser Zeit steigt die Temperatur auf 35°C und nach weiteren 45 Minuten auf 40°C an. Bei dieser Temperatur wird die Trimerisationsreaktion aufrechterhalten. Nach 6 Stunden ist ein NCO-Gehalt von 40.5 % erreicht. Das Reaktionsprodukt wird mit 0,3 ml Nonafluorbutansulfonsäure in 1 ml Dimethylformamid stabilisiert und anschließend im Hochvakuum gedünnschichtet.
Ausbeute: 417 g (31 %)
Jod-Farbzahl (DIN 6162): 3
NCO-Gehalt: 22,0 %
Viskosität (25°C): 3100 mPa.s
monomeres HDI: 0,18 %

Polyisocyanat II

(Isocyanuratgruppen aufweisendes Polyisocyanat auf Basis von 3-Isocyanatomethyl-3,5,5-trimethyl-5-isocyanatocyclohexan (IPDI))

4

1332 g IPDI werden auf 80°C erwärmt. Innerhalb von 45 Minuten werden 15 ml einer 6 %igen Lösung von 2-Hydroxyethyltrimethylammoniumhydroxid in Dimethylformamid/Methanol (4:1) langsam und gleichmäßig zugetropft. Hierbei steigt die Temperatur auf ca. 88°C (90°C sollten nicht überschritten werden; bei zu hoher Temperatur verläuft die Trimerisierung unspezifisch und führt zu höheren Viskositäten des Endproduktes). Es wird nach Beendigung des Zutropfens eine halbe Stunde nachgerührt, wobei die Temperatur auf 80°C sinkt. Der NCO-Gehalt der Trimerisatlösung liegt hiernach bei 30,6 %. Im Hochvakuum wird gedünnschichtet und das Harz anschließend 75 %ig in Ethylglykolacetat gelöst.

Ausbeute (Harz): 580 g (44 %)

Viskosität (Lösung): 5107 mPa.s (25°C)

NCO-Gehalt (Lösung): 12,5 %

freies IPDI (Lösung): 0,18 %

Polyesterpolyole I bis III

Die in Tabelle 1 angegebenen Ausgangsmaterialien werden gemischt und stufenweise unter Inertgas auf 210°C erhitzt. Die Veresterungsreaktion wird unter Abdestillieren des Reaktionswassers bei dieser Temperatur solange fortgesetzt, bis eine Säurezahl kleiner 5 mg KOH/g erreicht ist.

Man erhält farblose, bis schwach gelbliche Weichharze, die in üblichen Lösemitteln klar löslich sind.

Tabelle 1

|  | Polyesterpolyol | | |
|---|---|---|---|
|  | I | II | III |
| Einwaage (%): | | | |
| Adipinsäure | 13,2 | 10,2 | 42,2 |
| Phthalsäureanhydrid | 6,6 | 5,0 | |
| Isophthalsäure | 37,6 | 28,9 | |
| Hexandiol-1,6 | 42,7 | 65,3 | 68,2 |
| Trimethylolpropan | 12,2 | | |
| Kenndaten: | | | |
| Säurezahl (mg KOH/g) | 2 | 4 | 2 |
| OH-Zah (mg KOH/g) | 140 | 250 | 330 |

Beispiele 1 bis 3 (erfindungsgemäßes Verfahren) und 4 bis 6 (Vergleichsbeispiele)

In allen Beispielen wird die Reaktion in Gegenwart von 0,05 % Hydrochinon und 0,02 % Dibutylzinndilaurat und unter Luftdurchleiten durchgeführt. Die Viskositätsangaben beziehen sich auf Messungen im Rheometer bei 23°C.

Die in Tabelle 2 aufgeführten Polyisocyanate werden in Butylacetat gelöst und auf 60°C erhitzt. Daraufhin wird Hydroxyethylacrylat so zugetropft, daß die Temperatur nicht über 70°C ansteigt. Anschließend werden die jeweiligen Polyesterpolyole zugegeben und die Mischung bei 60°C solange gehalten, bis der NCO-Gehalt auf 0 gefallen ist. Das Butylacetat wird hierbei in einer solchen Menge eingesetzt, daß die in der nachstehenden Tabelle 2 genannten Festgehalte, die sich auf die Gesamteinwaage beziehen, resultieren.

EP 0 315 020 B1

Tabelle 2

| | Beispiele | | | Vergleichsbeispiele | | |
|---|---|---|---|---|---|---|
| | 1 | 2 | 3 | 4 | 5 | 6 |
| Einwaage (%): | | | | | | |
| Polyisocyanat I | 59,8 | 66,7 | 66,4 | 67,6 | 64,4 | |
| Polyisocyanat II | | | | | | 72,1 |
| Hydroxyethylacrylat | 30,2 | 24,5 | 22,6 | 27,4 | 35,6 | 21,0 |
| Hexandiol-1,6 | | | | 5,0 | | |
| Polyesterpolyol I | 10,0 | | | | | 6,9 |
| Polyesterpolyol II | | 8,8 | | | | |
| Polyesterpolyol III | | | 11,0 | | | |
| Kenndaten: | | | | | | |
| Festgehalt | 80 | 80 | 80 | 80 | 70 | 70 |
| Viskosität (mPa.s/25°C) | 9000 | 18000 | 11000 | 25000 | 2000 | 32000 |

Verwendungsbeispiele 1 bis 3 (erfindungsgemäß) und 4 bis 6 (Vergleichsbeispiele)

Die Produkte der vorstehenden Beispiele 1 bis 6 werden mit 1,5 % tert.-Butylperbenzoat und 1 % Kobaltoctoat (2,2 % Metallgehalt) versetzt. Die Standzeiten dieser Lacke beträgt mehr als 2 Tage. Nach Auftragen der Lackfilme auf Stahlbleche werden sie 40 Minuten bei 100°C erhitzt. Die resultierenden, trockenen Lackschichten weisen eine Dicke von ca. 50 $\mu$m auf.

In Tabelle 3 sind die erhaltenen Filmeigenschaften aufgeführt.

## Tabelle 3

| | Verwendungs-beispiele | | | Vergleichs-beispiele | | |
|---|---|---|---|---|---|---|
| | 1 | 2 | 3 | 4 | 5 | 6 |
| Pendelhärte (sec.)[a] | 135 | 144 | 127 | 170 | 190 | 200 |
| Dehnbarkeit (mm)[b] | 5,0 | 5,5 | 5,5 | 3,0 | 1,0 | 0,4 |
| Lösemittel-festigkeit[c] | gut | gut | gut | gut | gut | schlecht |

a) Die Pendelhärten werden nach König (DIN 53 157) bestimmt.

6

b) Die Dehnbarkeit der Beschichtung wird durch Tiefungs-prüfung nach Erichsen (DIN 53 156) bestimmt. Eine Dehnbarkeit $\leq$ 3 mm ist für eine sinnvolle Anwendung nicht ausreichend.

c) Zur Bestimmung der Lösemittelfestigkeit wird ein mit Lösemittel getränkter Wattebausch 1 Minute lang auf den Lackfilm gelegt. Danach wird durch Ankratzen mit dem Fingernagel die Filmveränderung beurteilt. Als Lösemittel wird Toluol, Methoxypropylacetat, Ethyl-acetat und Aceton verwendet.

Außer bei Vergleichsbeispiel 6 sind die Oberflächen der Lackfilme gar nicht oder nur schwer ankratzbar. Während mit den erfindungsgemäßen Produkten der Beispiele 1 bis 3 sowohl harte als auch elastische und lösemittelfeste Beschichtungen entstehen, zeigen die Produkte der Vergleichsbeispiele 4 bis 6 keine ausreichende Eigenschaften.

Verwendungsbeispiel 7 (Fotochemische Härtung)

100 Gew.-Teile des Produkte des Herstellungsbeispiels 1 werden mit 65 Gew.-Teilen Butylacetat und 0,5 Gew.-Teilen 2-Hydroxy-2-methyl-1-phenyl-propan-1-on (Fotoinitiator) versetzt und auf eine furnierte Spanplatte so aufgetragen, daß nach dem Abdunsten des Lösemittels ein Trockenauftrag von 30 $\mu$m erreicht wird.

Die Platte wird mit einer Geschwindigkeit von 20 m/min. unter einem Hanoviastrahler (80 W/cm, 10 cm Abstand) durchbewegt. Es entsteht ein harter, elastischer, kratz-, wasser- und chemikalienfester Überzug.

**Patentansprüche**

1. Verfahren zur Herstellung von Isocyanuratgruppen und olefinische Doppelbindungen aufweisenden Verbindungen durch Umsetzung von

   a) einer, Isocyanuratgruppen aufweisende Polyisocyanate enthaltenden, Polyisocyanat-Komponente mit

   b) einer olefinisch ungesättigten Alkoholkomponente, bestehend aus mindestens einem Hydroxyalkylester der Acrylsäure oder der Methacrylsäure, dadurch gekennzeichnet, daß man

   a) als Polyisocyanatkomponente (i) gegebenenfalls im Gemisch mit seinen höheren, mehr als einem Isocyanuratring aufweisenden Homologen vorliegendes N,N′,N″-Tris(isocyanatohexyl)-isocyanurat oder (ii) Gemische der unter (i) genannten Polyisocyanate mit bis zu 40 NCO-Äquivalent-%, bezogen auf die gesamte Komponente a) an anderen Polyisocyanaten mit aliphatisch und/oder cycloaliphatisch gebundenen Isocyanatgruppen

   verwendet und die Umsetzung unter Mitverwendung von

   c) einer Polyolkomponente, bestehend im wesentlichen aus einem Polyesterpolyol der OH-Zahl 80 bis 350 auf Basis von (i) einer Säurekomponente, bestehend zu mindestens 80 Carboxyl-Äquivalent-% aus Adipinsäure und/oder Isophthalsäure und (ii) einer Polyolkomponente, bestehend zumindest zu 70 Hydroxyläquivalent-% aus 1,6-Hexandiol

   durchführt, wobei die Menge der Komponente c) 20 bis 150 Gew.-%, bezogen auf das Gewicht der Komponente b), ausmacht und die Umsetzung unter Einhaltung eines NCO/OH-Äquivalentverhältnisses von 0,9:1 bis 1,1:1 durchführt, wobei die alkoholischen Komponenten b) und c) mit der Polyisocyanatkomponente in beliebiger Reihenfolge oder in Abmischung umgesetzt werden.

2. Verfahren gemäß Anspruch 1, dadurch gekennzeichnet, daß man als Komponente b) 2-Hydroxyethylacrylat, 2-Hydroxyethylmethacrylat, 2-Hydroxypropylacrylat, 2-Hydroxypropylmethacrylat oder beliebige Gemische dieser Verbindungen verwendet.

7

**3.** Verfahren gemäß Anspruch 1 und 2, dadurch gekennzeichnet, daß man als Komponente c) ein Polyesterdiol aus Adipinsäure und 1,6-Hexandiol verwendet.

**4.** Verwendung der gemäß Anspruch 1 bis 3 erhältlichen Isocyanuratgruppen und olefinische Doppelbindungen aufweisenden Verbindungen als Bindemittel für unter dem Einfluß von Strahlen oder von Peroxiden in Gegenwart von Luftsauerstoff härtbaren Beschichtungsmitteln.

**Claims**

**1.** A process for the production of compounds containing isocyanurate groups and olefinic double bonds by reaction of

a) a polyisocyanate component containing isocyanurate polyisocyanates

with

b) an olefinically unsaturated alcohol component consisting of at least one hydroxylalkyl ester of acrylic acid or methacrylic acid,

characterized in that

a) the polyisocyanate component is (i) N,N',N"-tris-(isocyanatohexyl)-isocyanurate optionally present in admixture with its higher homologs containing more than one isocyanurate ring or (ii) mixtures of the polyisocyanates mentioned under (i) with up to 40 NCO-equivalent-%, based on the total component a), of other polyisocyanates containing aliphatically and/or cycloaliphatically bound isocyanate groups

and the reaction is carried out using

c) a polyol component consisting essentially of a polyester polyol having an OH value of 80 to 350 based on (i) an acid component, of which at least 80 carboxyl equivalent-% consists of adipic acid and/or isophthalic acid and (ii) a polyol component of which at least 70 hydroxyl equivalent-% consists of hexane-1,6-diol,

component c) making up 20 to 150% by weight, based on the weight of component b), and the reaction being carried out at an NCO:OH equivalent ratio maintained at 0.9:1 to 1.1:1, the alcoholic components b) and c) being reacted with the polyisocyanate component in any order or in admixture.

**2.** A process as claimed in claim 1, characterized in that component b) is 2-hydroxyethyl acrylate, 2-hydroxyethyl methacrylate, 2-hydroxypropyl acrylate, 2-hydroxypropyl methacrylate or a mixture of these compounds.

**3.** A process as claimed in claims 1 and 2, characterized in that a polyester diol of adipic acid and 1,6-hexanediol is used as component c).

**4.** The use of the compounds containing isocyanurate groups and olefinic double bonds obtainable by the process claimed in claims 1 to 3 as binders for coating compositions hardening under the effect of radiation or peroxides in the presence of air.

**Revendications**

**1.** Procédé de production de composés porteurs de groupes isocyanurate et de doubles liaisons oléfiniques par réaction

a) d'un composant polyisocyanate contenant des polyisocyanates porteurs de groupes isocyanurate

avec

b) un composant alcool à non-saturation oléfinique constitué d'au moins un ester hydroxyalkylique d'acide acrylique ou d'acide méthacrylique, caractérisé en ce qu'on utilise

a) comme composant polyisocyanate (i), le N,N',N"-tris-(isocyanatohexyl)-isocyanurate le cas échéant en mélange avec ses homologues supérieurs présentant plus d'un noyau isocyanurate ou (ii) des mélanges des polyisocyanates mentionnés en (i) avec jusqu'à 40 équivalents de NCO %, par rapport au composant a) total, d'autres polyisocyanates porteurs de groupes isocyanate en liaison aliphatique et/ou cycloaliphatique

et on conduit la réaction en utilisant simultanément

c) un composant polyol, constitué principalement d'un polyester-polyol d'indice d'hydroxyle égal à 80-350 à base (i) d'un composant acide constitué en proportion d'au moins 80 équivalents de carboxyle % d'acide adipique et/ou d'acide isophtalique et (ii) d'un composant polyol, constitué en proportion d'au moins 70 équivalents d'hydroxyle % de 1,6-hexanediol,
la quantité de composant c) étant de 20 à 150 % en poids par rapport au poids du composant b) et la réaction étant conduite avec maintien d'un rapport des équivalents de NCO aux équivalents de OH de 0,9:1 à 1,1:1, les composants alcooliques b) et c) étant mis en réaction avec le composant polyisocyanate dans un ordre quelconque ou en mélange.

2. Procédé suivant la revendication 1, caractérisé en ce qu'on utilise comme composant b) l'acrylate de 2-hydroxyéthyle, le méthacrylate de 2-hydroxyéthyle, l'acrylate de 2-hydroxypropyle, le méthacrylate de 2-hydroxypropyle ou des mélanges quelconques de ces composés.

3. Procédé suivant les revendications 1 et 2, caractérisé en ce qu'on utilise comme composant c) un polyesterdiol d'acide adipique et de 1,6-hexanediol.

4. Utilisation des composés porteurs de groupes isocyanurate et de doubles liaisons oléfiniques obtenus conformément aux revendications 1 à 3 comme liants pour des compositions de revêtement durcissables sous l'influence de rayons ou de peroxydes en présence de l'oxygène de l'air.